# FASCICULE DE BREVET EUROPEEN

(11) **EP 2 243 821 B1**
(45) Date de publication et mention de la délivrance du brevet: **26.11.2014**
(21) Numéro de dépôt: 09290293.1
(22) Date de dépôt: 20.04.2009
(51) Int. Cl.: C11D 3/386, C11D 3/00, A61L 2/18, B65B 5/00, C11D 7/42, A61L 12/14, A61L 2/16

(54) **Produit et procédé d'élimination des biofilms**
Biofilmbeseitigungsprodukt und -verfahren
Product and method for eliminating biofilms

(43) Date de publication de la demande: 27.10.2010
(73) Titulaire: Realco SA, 1348 Louvain La Neuve (BE); Institut National de la Recherche Agronomique (INRA), 75338 Paris Cedex 07 (FR)
(72) Inventeur: Boels, Gauthier, 1070 Bruxelles (BE); Blackman, Gordon, 1380 Lasnes (BE); Faille, Christine, 59000 Lille (FR); Lequette, Yannick, 62144 Mont-Saint-Eloi (FR); Clarisse, Martine, 59390 Lys-lez-Lannoy (FR)
(74) Mandataire: Coulon, Ludivine

(56) Documents cités:
- WO-A-92/13807
- US-A1- 2003 205 247
- Trudi M. Foreman ET AL: "Effects of charged water-soluble polymers on the stability and activity of yeast alcohol dehydrogenase and subtilisin Carlsberg", Biotechnology and Bioengineering, vol. 76, no. 3, 1 November 2001 (2001-11-01), pages 241-246, XP055126700, ISSN: 0006-3592, DOI: 10.1002/bit.1187

## Description

### Domaine technique

L'invention se rapporte au domaine de l'élimination des biofilms. Plus particulièrement, l'invention se rapporte à une composition et à un procédé pour éliminer des biofilms.

Le document WO92/13807 divulgue aussi une composition pour le traitement enzymatique de biofilms.

### Description de l'état de la technique

L'hygiène prend une importance croissante dans l'industrie alimentaire, dans les hôpitaux, et en particulier dans la sphère chirurgicale, dans les installations de potabilisation et de désalinisation d'eau, dans le traitement des eaux de process, et en particulier dans les eaux utilisées dans les tours de refroidissement et dans les nécessités de la vie courante, par exemple les lentilles de contact. On observe fréquemment que, lors de la circulation d'eau sur un support, des microorganismes circulant librement dans l'eau peuvent adhérer à la surface. Ces microorganismes peuvent alors développer une matrice extracellulaire adhésive composée de substances polymériques. Une communauté de microorganismes adhérés à une surface et englobée dans une telle matrice est appelée un biofilm. On observe malheureusement que cette matrice est très résistante, et peut constituer une barrière pour des agents qui agiraient contre les microorganismes. Les traitements classiques à base de soude et/ou comportant différents biocides n'agissent pas de manière suffisamment efficace car ils ne pénètrent pas le biofilm sur toute son épaisseur ou sont inhibés par certaines des molécules composant cette matrice. Le traitement n'est alors que partiellement efficace sur la surface supérieure du biofilm. En outre, ce dernier peut également piéger d'autres microorganismes, pathogènes notamment, que celui qui s'est installé initialement.

On connaît par WO98/26807 un procédé de traitement enzymatique d'un film biologique. Dans ce procédé, on met en contact le biofilm avec une composition de nettoyage comportant une ou plusieurs hydrolases pour éliminer ou libérer la couche de biofilm de la surface. Dans une seconde étape le biofilm est mis en contact avec une composition désinfectante bactéricide pour détruire les cellules bactériennes présentes dans le film. Cependant, l'utilisation simultanée de ces deux compositions est à l'origine d'un degré d'inactivation de certaines enzymes dans le mélange final. La rapidité et l'efficacité du nettoyage peuvent donc être améliorées en utilisant une composition où l'inactivation des enzymes est absente.

On connaît par US2003/0205247 une utilisation de solutions aqueuses contenant des enzymes pour le nettoyage de cuves de stockage ou de fermentation, contenant une ou plusieurs enzymes choisies parmi les suivantes : laccases, peroxydases, oxydoréductases, transférases, isomérases, lyases ou ligases. Le domaine d'action des solutions précitées est relativement étroit, propre à la brasserie. Ces solutions ne conviennent pas pour le nettoyage d'une large gamme de biofilm.

Il existe donc un besoin pour une composition et un procédé capable d'éliminer des biofilms, qui soient efficaces dans un délai raisonnable, agissent sur une large classe de biofilms produits par une large classe de microorganismes ou de groupes de microorganismes, qui ne soient pas nuisibles pour le support du biofilm, et agissent tant pour prévenir le développement de biofilms que sur des biofilms constitués depuis longtemps, et ayant atteint un stade de cohésion et de résistance importants.

### Résume de l'invention

La présente invention apporte une solution à un, plusieurs, ou la totalité des inconvénients des techniques conventionnelles et peut offrir d'autres avantages non envisagés avec les compositions et procédés connus.

L'invention se rapporte à une composition pour l'élimination des biofilms présents sur un substrat. Ladite composition est caractérisée en ce qu'elle comporte un composant détergent contenant un séquestrant et un composant enzymatique contenant au moins une protéase et au moins une laccase, ladite composition étant caractérisée en ce que ledit composant détergent contient en outre un mouillant et un dispersant et en ce que ledit composant enzymatique comporte en outre au moins une polysaccharidase. La constitution d'une composition comprenant un composant détergent et un composant enzymatique selon l'invention permet, de manière surprenante, d'améliorer significativement la rapidité et l'efficacité de l'élimination d'un biofilm. Cette composition permet d'éliminer la totalité ou la quasi-totalité du biofilm. En outre, la composition peut agir sur des biofilms même matures ou ayant un cycle de développement plus précoce et développés par de multiples espèces ou microorganismes différents. La combinaison d'un composant détergent et d'un composant enzymatique selon l'invention permet donc d'agir efficacement sur un biofilm. Le détergent élimine une partie superficielle du biofilm et mouille et/ou gonfle les structures organiques du biofilm favorisant l'accessibilité du composant enzymatique qui fragilise et dégrade la matrice du biofilm. Cette action combinée favorise l'accessibilité à la composition des couches plus profondes, permettant un détachement optimal du biofilm tout en préservant le substrat.

Selon un mode de réalisation préféré de l'invention, ladite composition peut être une solution ayant un pH compris approximativement entre 8 et 11, préférentiellement compris approximativement entre 9,5 et 10,5 et plus préférentiellement compris approximativement entre 9,5 et 10. La valeur du pH de ladite composition influence de manière importante son efficacité vis-à-vis du biofilm. Une solution de ladite composition dont le pH est compris approximativement entre 8 et 11 permet donc d'éliminer de manière surprenante la totalité ou la quasi-totalité du biofilm. Alternativement, ladite composition peut être sous forme solide puis être dissoute avant utilisation dans un solvant afin d'obtenir une solution dont le pH est compris approximativement entre 8 et 11.

Préférentiellement, le composant enzymatique comprend une proportion en protéase comprise entre 10 et 50%, une proportion de laccase comprise entre 5 et 35% et une proportion de polysaccharidase comprise entre 5 et 20%. Selon un mode de réalisation préféré de l'invention, le composant enzymatique peut contenir entre 1 et 10 protéases, préférentiellement entre 1 et 5 protéases, plus préférentiellement peut contenir 2, 3, 4 ou 5 protéases.

Des exemples non limitatifs d'enzymes protéases appartenant à la classe EC 3.4 et susceptible d'être utilisées dans l'invention sont les amino-peptidases (EC 3.4.11), les dipeptidases (EC 3.4.13), les dipeptidyl-peptidases et tripeptidylpeptidases (EC 3.4.14), les peptidyl-dipeptidases (EC 3.4.15), les sérine carboxypeptidases (EC 3.4.16), les métallo carboxypeptidases (EC 3.4.17), les cystéine carboxypeptidases (EC 3.4.18), les oméga peptidases (EC 3.4.19), les sérine endopeptidases (EC 3.4.21), les cystéine endopeptidases (EC 3.4.22), les aspartique endopeptidases (EC 3.4.23), les métallo endopeptidases (EC 3.4.24), les thréonine endopeptidases (EC 3.4.25), et les endopeptidases appartenant à la classe EC 3.4.99. Préférentiellement, les protéases appartiennent à la classe EC 3.4.21. Les protéases sont disponibles commercialement et sous différentes formes incluant les poudres, les granulés, les suspensions, les solutions liquides. Les laccases utilisées dans l'invention appartiennent à la classe EC 1.10.3.2. Les laccases sont des enzymes contenant du cuivre et ont pour fonction d'oxyder un substrat en présence d'oxygène. Plus spécifiquement, les laccases sont des oxydoréductases qui fonctionnent avec l'oxygène moléculaire comme accepteur d'électrons. Ladite au moins une polysaccharidase utilisée dans l'invention est une enzyme ayant pour fonction de briser des liaisons au sein des polysaccharides. Préférentiellement, ladite au moins une polysaccharidase peut être une alpha-amylase, cellulase, hemi-cellulase, glucosidase, beta-glucanase ou pectinase. Plus préférentiellement, ladite au moins une polysaccharidase peut être une alpha-amylase appartenant à la classe EC 3.2.1.1 ayant pour fonction de briser des liens (1-4)-alpha-glycosidiques dans des polysaccharides contenant trois unités ou plus alpha-(1-4)-D-glucose.

Préférentiellement, le composant enzymatique peut comprendre une proportion en laccase approximativement de 30%, une proportion en protéase approximativement de 30%, une proportion en alpha-amylase approximativement de 10%. Selon un autre mode de réalisation préféré, si le composant enzymatique comprend 2 protéases, la proportion en laccase peut être approximativement de 30%, la proportion totale en protéases approximativement de 30%, la proportion en alpha-amylase approximativement de 10%. Selon un autre mode de réalisation préféré, si le composant enzymatique comprend 2 protéases, la proportion en laccase peut être de 30%, la proportion totale en protéases de 30%, la proportion en alpha-amylase de 10%. Par exemple, le rapport entre chaque protéases peut être compris entre 1:2 et 2:1, préférentiellement le rapport entre chaque protéases peut être de 1:1. Les enzymes présentes dans le composant enzymatique ont une action complémentaire sur le biofilm. Par exemple, la laccase présente une grande efficacité sur les souillures non attaquées par l'alpha-amylase ou les protéases.

Selon un mode de réalisation préféré de l'invention, le composant enzymatique peut être une solution ou sous forme solide. Préférentiellement, le composant enzymatique est une solution dont le pH peut être compris entre 8 et 10. Préférentiellement, le composant enzymatique est une solution aqueuse dont le pH peut être compris entre 8,5 et 9,5 ; plus préférentiellement le pH peut être approximativement de 9,0. Alternativement, le composant enzymatique peut être sous forme solide telle que par exemple d'un lyophilisat, de poudres, de granulés ou tout autre forme permettant la solubilisation dudit composant dans un solvant, puis dissous dans un solvant. Le solvant peut être de l'eau ou une solution aqueuse, acide, basique ou neutre. La solution aqueuse peut contenir par exemple un ou plusieurs composés tels que par exemple des détergents.

Le composant détergent comprend un séquestrant, un dispersant et un mouillant. Selon un mode de réalisation préféré de l'invention, le composant détergent peut être une solution ou sous forme solide. Préférentiellement, le composant détergent est une solution aqueuse dont le pH est compris entre 11,0 et 14,0, plus préférentiellement compris entre 12,0 et 14,0, le plus préférentiellement entre 12,8 et 13,8. Préférentiellement, le composant détergent comprend une proportion de séquestrant comprise entre 1 et 10%, une proportion de dispersant comprise entre 1 et 10% et une proportion de mouillant comprise entre 1 et 15%. Le séquestrant est une substance chimique ayant la capacité à former des complexes avec des ions minéraux qu'il fixe sous une forme empêchant leur précipitation par les réactions habituelles. A titre d'exemple, le séquestrant peut être l'acide éthylène-diamine-tétraacétique, le glucono-delta-lactone, le gluconate de sodium, le gluconate de potassium, le gluconate de calcium, l'acide citrique, l'acide phosphorique, l'acide tartrique, l'acétate de sodium, le sorbitol, un composé comportant un atome de phosphore. Préférentiellement, le séquestrant peut être un oxyde de phosphore tel que phosphonate, phosphinate ou phosphate, ou un sel de celui-ci, une amine ou un oxyde d'amine portant au moins, dans sa structure, un groupement fonctionnel phosphine, oxyde de phosphine, phophinite, phosphonite, phosphite, phosphonate, phosphinate ou phosphate, ou un sel de ceux-ci. Plus préférentiellement le séquestrant peut être un phosphonate ou un sel de celui-ci, une amine ou un oxyde d'amine comportant au moins, dans sa structure, un groupement fonctionnel phosphine, oxyde de phosphine, phosphinite, phosphonite, phosphite, phosphonate, phosphinate ou phosphate, ou un sel de ceux-ci. A titre d'exemple non limitatif, le phosphonate peut être de formule générale R¹(R²O)(R³O)P=O dans lequel R¹, R² et R³ sont indépendamment sélectionnés parmi le groupe hydrogène, alkyle, alkyle substitué, alkyle-amino substitué ou non, aminoalkyl substitué ou non, aryle ou aryle substitué. A titre d'exemple non limitatif, l'amine ou l'oxyde d'amine peuvent comporter un, deux ou trois substituant(s) de formule générale CR⁴R⁵W dans laquelle R⁴ et R⁵ sont indépendamment sélectionnés parmi le groupe hydrogène, alkyle, alkyle substitué, alkyle-amino substitué ou non, aminoalkyl substitué ou non, aryle ou aryle substitué, et W est sélectionné parmi le groupe phosphonate, phosphinate ou phosphate. Le séquestrant peut être sous la forme d'un sel de sodium, calcium, lithium, magnésium ou potassium ; préférentiellement, le séquestrant peut être sous la forme d'un sel de sodium, calcium, ou potassium. Le dispersant est une substance chimique ayant la capacité à améliorer la séparation des particules d'une suspension afin de prévenir l'agglutination, l'agrégation et/ou la décantation. Le dispersant peut être un polymère soluble ou partiellement soluble dans l'eau tel que par exemple le polyéthylène glycol, les dérivés de la cellulose ou un polymère comprenant au moins un motif acide acrylique ou ester acrylique. Préférentiellement, le dispersant est un polymère comprenant au moins un motif acide acrylique ou ester acrylique de formule générale -(CH₂-CH-COOR)- dans lequel R peut être un hydrogène, un alkyle ou un alkyle substitué, un aryle ou un aryle substitué. En particulier le dispersant est un polymère ayant une masse moléculaire Mw comprise entre 500 et 10000. Plus préférentiellement, le dispersant est un homopolymère de l'acide acrylique. En particulier, le dispersant peut être un homopolymère de l'acide acrylique ayant une masse moléculaire comprise entre 2000 et 6000. Le mouillant est une substance chimique amphiphile, ou une composition comprenant ladite substance chimique amphiphile, qui modifie la tension superficielle entre deux surfaces. Le mouillant a pour avantage de favoriser l'étalement d'un liquide sur un solide. Le mouillant peut être anionique, cationique, non-ionique ou zwitterionique. Préférentiellement, le mouillant peut être un mouillant anionique ou non-ionique, c'est-à-dire que la partie hydrophile est chargée négativement ou ne comporte aucune charge nette, ou peut être une composition comprenant un mouillant anionique. Plus particulièrement, le mouillant peut être le Tween, un ester de saccharose ou une composition comprenant un alkyle sulfate de sodium et un alcool.

L'invention se rapporte à une méthode d'élimination des biofilms présents sur un substrat caractérisée en ce qu'elle comporte les étapes suivantes de :
a) mise à disposition d'un composant détergent contenant un séquestrant, un dispersant et un mouillant; et d'un composant enzymatique contenant au moins une protéase, au moins une laccase, et au moins une polysaccharidase,
b) mise en solution ou dilution du composant détergent dans l'eau,
c) mise en solution du composant enzymatique dans la solution formée à l'étape b) pour former la solution de ladite composition selon l'invention,
   ou b') mise en solution ou dilution du composant enzymatique dans l'eau,
c') mise en solution du composant détergent dans la solution formée à l'étape
b') pour former la solution de ladite composition selon l'invention,
d) application de la solution de ladite la composition formée à l'étape c) ou c') sur le substrat pendant une période de 15 minutes à 4 heures.
Alternativement, les étapes b) et c), ou b') et c'), peuvent être effectuées de manière simultanée pour former une solution de ladite composition selon l'invention.

Préférentiellement, la méthode comporte les étapes suivantes de :
a) mise à disposition d'un composant détergent contenant un séquestrant, un dispersant et un mouillant; et d'un composant enzymatique contenant au moins une protéase, au moins une laccase, et au moins une polysaccharidase,
b) mise en solution ou dilution du composant détergent dans l'eau,
c) mise en solution du composant enzymatique dans la solution formée à l'étape b) pour former la solution de ladite composition selon l'invention,
d) application de la solution de ladite la composition formée à l'étape c) sur le substrat pendant une période de 15 minutes à 4 heures.

Selon un mode de réalisation préféré de l'invention, le pH de la solution formée lors de l'étape a) est compris approximativement entre 11,0 et 14,0, préférentiellement compris approximativement entre 12,0 et 14,0 et plus préférentiellement compris entre 12,8 et 13,8. De préférence, la température de la solution du composant détergent formée lors de l'étape a) peut être comprise entre approximativement 35°C et 50°C.

Selon un mode de réalisation préféré de l'invention, la solution de ladite composition formée à l'étape c), a un pH compris approximativement entre 8 et 11, préférentiellement compris approximativement entre 9,5 et 10,5 et plus préférentiellement compris entre 9,5 et 10.

Selon un mode de réalisation préféré de l'invention, la composition selon l'invention est appliquée sur un substrat couvert d'un biofilm pendant approximativement 30 à 50 minutes.

Préférentiellement, le composant détergent comprend une proportion de séquestrant comprise entre 1 et 10%, une proportion de dispersant comprise entre 1 et 10% et une proportion de mouillant comprise entre 1 et 15%. Préférentiellement, le composant enzymatique comprend une proportion en protéase comprise entre 10 et 50%, une proportion de laccase comprise entre 5 et 35% et une proportion de polysaccharidase comprise entre 5 et 20%. Plus préférentiellement, la au moins une polysaccharidase peut être une alpha-amylase.

La présente méthode permet d'éliminer efficacement la totalité ou la quasi-totalité du biofilm, ne laissant alors sur le substrat que des cellules isolées sans protection de la matrice. L'action postérieure d'un biocide permet de détruire la souche microbienne. Une phase de désinfection ultérieure sera donc beaucoup plus efficace suite à l'application d'une solution de la composition selon l'invention que suite à l'application d'une phase de nettoyage ne permettant pas cette élimination totale de la matrice. Ainsi, selon un mode de réalisation préféré de l'invention, la méthode comprend en outre une étape ultérieure d'application d'un biocide. Par exemple, les biocides peuvent être, de manière non limitative, de type oxydant tel que l'acide peracétique, le peroxyde d'hydrogène, le monopersulfate de potassium, l'hypochlorite de soude. Selon l'invention, l'application d'un biocide doit être postérieure à l'application de la composition selon l'invention pour éviter la désactivation des enzymes présentes dans ladite composition par les biocides.

L'invention comporte également l'utilisation de la composition suivant l'invention pour l'élimination de biofilms sur un substrat. La composition peut s'utiliser dans des installations fermées ou par trempage. Plus particulièrement, l'invention se rapporte à l'utilisation de ladite composition pour le nettoyage des sols et des surfaces, pour le nettoyage en place ou le trempage. Le nettoyage par trempage est notamment utilisé pour nettoyer du matériel chirurgical, les lentilles de contact. La composition selon l'invention peut être utilisée pour le nettoyage de circuit d'eau technique et de process, les systèmes d'échangeurs de conditionnement d'air ou dans l'industrie alimentaire.

Selon un autre aspect, l'invention se rapporte à une trousse, destinée à l'élimination d'un biofilm sur un substrat, et caractérisée en ce qu'elle comprend au moins un échantillon d'un composant détergent en solution ou sous forme solide contenant un séquestrant, un dispersant et un mouillant, et au moins un échantillon d'un composant enzymatique en solution ou sous forme solide contenant au moins une protéase, au moins une laccase, et au moins une polysaccharidase.. Préférentiellement, l'échantillon du composant enzymatique peut comprendre une proportion en protéase comprise entre 10 et 50%, une proportion de laccase comprise entre 5 et 35% et une proportion de polysaccharidase comprise entre 5 et 20%. Préférentiellement, ladite au moins une polysaccharidase peut être une alpha-amylase. Selon un mode de réalisation préféré de l'invention, l'échantillon du composant enzymatique peut contenir entre 1 et 10 protéases, préférentiellement entre 1 et 5 protéases, plus préférentiellement peut contenir 1, 2, 3, 4 ou 5 protéases. Selon un autre mode de réalisation préféré, l'échantillon du composant enzymatique contenu dans la trousse peut comprendre 2 protéases. Préférentiellement, si l'échantillon du composant enzymatique comprend 2 protéases, la proportion en laccase peut être approximativement de 30%, la proportion totale en protéases peut être approximativement de 30%, la proportion en alpha-amylase peut être approximativement de 10%. Préférentiellement, si l'échantillon du composant enzymatique comprend 2 protéases, la proportion en laccase peut être de 30%, la proportion totale en protéases peut être de 30%, la proportion en alpha-amylase peut être de 10%. Par exemple, le rapport entre chaque protéase peut être compris entre 1:2 et 2:1, préférentiellement le rapport entre chaque protéase peut être de 1:1. Si l'échantillon du composant enzymatique contenu dans la trousse est une solution, le pH de celle-ci peut être compris entre 8 et 10, préférentiellement, le pH peut être compris entre 8,5 et 9,5, plus préférentiellement le pH peut être approximativement de 9,0. Préférentiellement, l'échantillon du composant détergent comprend une proportion de séquestrant comprise approximativement entre 1 et 10%, une proportion de dispersant comprise approximativement entre 1 et 10% et une proportion de mouillant comprise approximativement entre 1 et 15%. A titre d'exemple, le séquestrant peut être l'acide éthylène-diamine-tétraacétique, le glucono-delta-lactone, le gluconate de sodium, le gluconate de potassium, le gluconate de calcium, l'acide citrique, l'acide phosphorique, l'acide tartrique, l'acétate de sodium, le sorbitol, un composé comportant un atome de phosphore. Préférentiellement, le séquestrant peut être un oxyde de phosphore tel que phosphonate, phosphinate ou phosphate, ou un sel de celui-ci, une amine ou un oxyde d'amine, ou un sel de ceux-ci portant au moins, dans sa structure, un groupement fonctionnel phosphine, oxyde de phosphine, phophinite, phosphonite, phosphite, phosphonate, phosphinate ou phosphate. Plus préférentiellement, le séquestrant peut être un phosphonate ou un sel de celui-ci, une amine ou un oxyde d'amine, ou un sel de ceux-ci, comportant au moins, dans sa structure, un groupement fonctionnel phosphine, oxyde de phosphine, phosphinite, phosphonite, phosphite, phosphonate, phosphinate ou phosphate. A titre d'exemple non limitatif, le phosphonate peut être de formule générale R¹(R²O)(R³O)P=O dans lequel R¹, R² et R³ sont indépendamment sélectionnés parmi le groupe hydrogène, alkyle, alkyle substitué, alkyle-amino substitué ou non, aminoalkyl substitué ou non, aryle ou aryle substitué. A titre d'exemple non limitatif, l'amine ou l'oxyde d'amine peuvent comporter un, deux ou trois substituant(s) de formule générale CR⁴R⁵W dans laquelle R⁴ et R⁵ sont indépendamment sélectionnés parmi le groupe hydrogène, alkyle, alkyle substitué, alkyle-amino substitué ou non, aminoalkyl substitué ou non, aryle ou aryle substitué, et W est sélectionné parmi le groupe phosphonate, phosphinate ou phosphate. Le séquestrant peut être sous la forme d'un sel de sodium, calcium, lithium, magnésium ou potassium ; préférentiellement, le séquestrant peut être sous la forme d'un sel de sodium, calcium, ou potassium. Le dispersant peut être un polymère soluble ou partiellement soluble dans l'eau tel que par exemple le polyéthylène glycol, les dérivés de la cellulose ou un polymère comprenant au moins un motif acide acrylique ou ester acrylique. Préférentiellement, le dispersant est un polymère comprenant au moins un motif acide acrylique ou ester acrylique de formule générale -(CH₂-CH-COOR)- dans lequel R peut être un hydrogène, un alkyle ou un alkyle substitué, un aryle ou un aryle substitué. En particulier le dispersant est un polymère ayant une masse moléculaire Mw comprise entre approximativement 500 et 10000. Plus préférentiellement, le dispersant est un homopolymère de l'acide acrylique. En particulier, le dispersant peut être un homopolymère de l'acide acrylique ayant une masse moléculaire comprise entre 2000 et 6000. Le mouillant peut être anionique, cationique, non-ionique ou zwitterionique. Préférentiellement, le mouillant peut être un mouillant anionique ou non-ionique, c'est-à-dire que la partie hydrophile est chargée négativement ou n'a pas de charge nette, ou peut être une composition comprenant un mouillant anionique. Plus particulièrement, le mouillant peut être une composition comprenant un alkyle sulfate de sodium et un alcool. Si l'échantillon du composant détergent est une solution, le pH de celle-ci est compris entre 11,0 et 14,0, plus préférentiellement compris entre 12,0 et 14,0, le plus préférentiellement entre 12,8 et 13,8.

### Brève description des dessins

La FIG. 1 représente un graphique montrant la quantité de cellules d'une souche de *Pseudomonas fluorescens* présente sur un substrat après l'application de différentes solutions de traitement.

La FIG. 2 représente un graphique montrant la quantité de cellules d'une souche *Bacillus mycoïdes* présente sur un substrat après l'application de différentes solutions de traitement.

La FIG. 3 représente un graphique montrant la quantité de cellules d'une souche *Bacillus cereus* présente sur un substrat après l'application de différentes solutions de traitement.

La FIG. 4 représente des images de la surface d'un substrat, prises par un microscope à épifluorescence, après l'application de différentes solutions de traitement sur ledit substrat recouvert par un biofilm de la souche de *Pseudomonas fluorescens*.

La FIG. 5 représente des images de la surface d'un substrat, prises par un microscope à épifluorescence, après l'application de différentes solutions de traitement sur ledit substrat recouvert par un biofilm de la souche de *Bacillus mycoïdes*.

La FIG. 6 représente des images de la surface d'un substrat, prises par un microscope à épifluorescence, après l'application de différentes solutions de traitement sur ledit substrat recouvert par un biofilm de la souche de *Bacillus cereus.*

### Description détaillée de l'invention

### Exemple 1

Description de la sélection des conditions de test : les tests sont effectués dans le cadre d'une procédure de nettoyage en place (NEP) sur un pilote industriel. Les biofilms sont développés sur une face plane d'un cylindre en inox perforé en son centre. Ce cylindre, une fois introduit dans une canalisation de diamètre intérieur égal au diamètre extérieur du cylindre, provoque un brusque rétrécissement du diamètre de celle-ci, forçant le liquide à passer par la perforation en son centre. Cette contrainte entraîne des perturbations du flux, créant ainsi des zones mortes à la surface du cylindre. Ces zones mortes sont favorables à la formation d'un biofilm et de plus défavorables à son arrachement mécanique par le flux. Elles représentent donc typiquement des zones difficiles pour l'élimination d'un biofilm. Trois souches distinctes sont utilisées : *Pseudomonas fluorescens* (fourni par University of Cornell, Department of Food science, Ithaca, New York, 14853, USA (Kathryn J. Boor, kjb4@cornell.edu)), *Bacillus mycoides* (fourni par le laboratoire AFSSA à Maison-Alfort, France (Brigitte Carpentier, b.carpentier@lerpac.afssa.fr)), *Bacillus cereus* (fourni par INRA-UR638, Laboratoire de Génie des Procédés et Technologies Alimentaires, Villeneuve d'Ascq, France (Christine Faille, Christine.faille@lille.inra.fr)).

Un milieu de croissance est préparé comme suit : des extraits de viande en poudre (Biokar) sont dissous dans l'eau distillée à 0,1% et sont stérilisés. 20 ml d'inoculum à 5.10⁷ CFU/ml dans une solution de Trypticase Soy broth (TSB, Biokar) sont déposés sur la surface des cylindres. Ces cylindres sont incubés en chambre humide à 30°C pendant 2 heures pour permettre l'adhésion des cellules. Ensuite la solution de TSB est retirée et remplacée par 20 ml du milieu de croissance viande et les cylindres sont incubés 24 h à 30°C. Le milieu de croissance est alors remplacé par du milieu frais à base viande et les cylindres sont à nouveau incubés pendant 24h. La procédure de nettoyage consiste à introduire les cylindres contaminés par le biofilm dans les canalisations droites d'un pilote industriel nettoyé par circulation (procédure de nettoyage en place - NEP). Les solutions de traitement sont circulées durant 30 minutes à 45°C avec un débit de 300 l/h Pour deux des souches, des essais ont été également réalisés avec un débit de 600l/h. Chaque essai est répété trois fois et une moyenne est établie. Le développement et l'élimination des biofilms sont suivis par observation microscopique et par quantification des cellules viables présentes sur les cylindres par dénombrement sur milieu Trypticase soy agar après décrochement des cellules.

Description du protocole de préparation de la composition
Le composant détergent est préparé en mélangeant dans un volume d'eau déterminé un phosphonate, un polyacrylate et un mouillant anionique. Les proportions respectives dans le composant détergent sont de 3%, 4% et 3%. Le pH de la solution est amené à 13,3 par dilution. Une solution du composant enzymatique est préparée. Celle-ci comprend une proportion de 30% de protéases (EC 3.4.21), 30% de laccase (EC 1.10.3.2) et 10% d'alpha-amylase (EC 3.2.1.1). Le pH de la solution du composant enzymatique est porté à 9 par ajout progressif d'une solution d'hydroxyde de potassium. La solution de la composition selon l'invention est préparée par l'ajout dans de l'eau du composant détergent et du composant enzymatique. La solution de ladite composition selon l'invention comprend 1% de composant détergent et 0,05% de composant enzymatique. Le pH de la solution de ladite composition selon l'invention était approximativement de 10.

### Description des tests et de leurs résultats

### Test avec la souche de Pseudomonas fluorescens

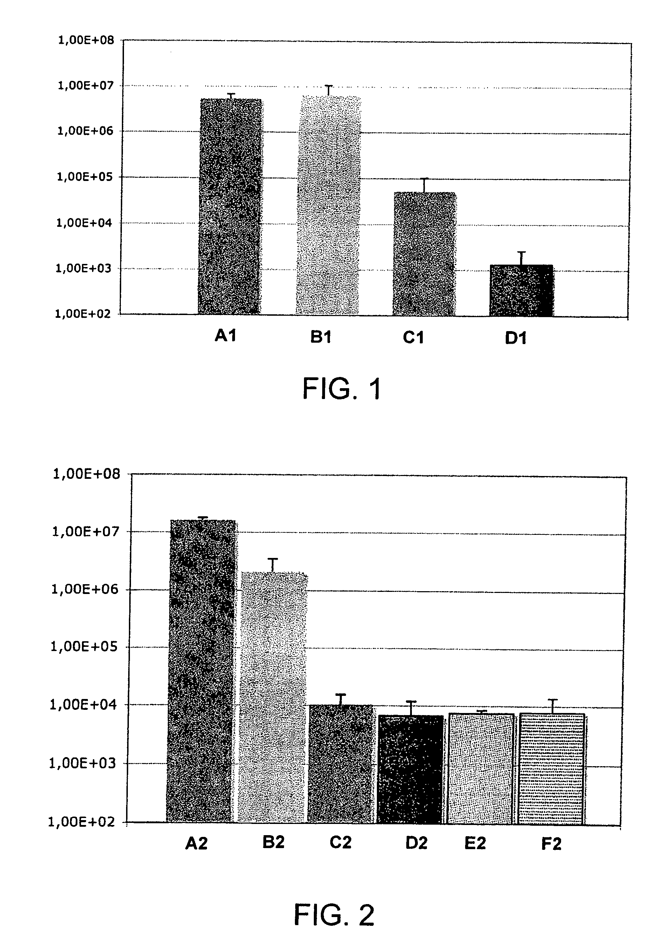

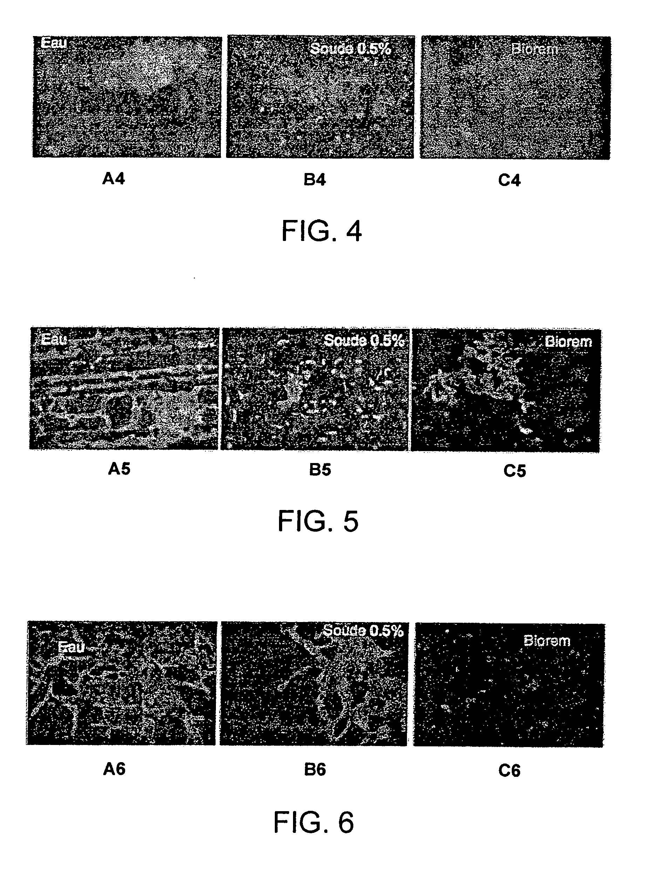

Le graphique représenté à la FIG. 1 montre la quantité de biofilm présente sur la paroi du cylindre après l'application de solutions de traitements suivant les conditions énoncées ci-dessus. Les surfaces du substrat ont été recouvertes d'un biofilm formé par la souche de *Pseudomonas fluorescens.* La quantité initiale de biofilm présente avant le traitement a été notée A1 dans la FIG. 1. Trois solutions ont été ensuite comparées : une solution comprenant uniquement de l'eau (B1), une solution de soude à 0,5% (C1) et une solution de la composition selon l'invention (D1). Les solutions ont été appliquées à un débit de 300 l/h et une température de 45°C. La solution (B1) comprenant de l'eau permet d'évaluer l'arrachement mécanique des biofilms par le flux. On observe ainsi que le biofilm a résisté à l'arrachement mécanique puisque les quantités de biofilm sont identiques (courbes A1 et B1). L'application d'une solution de la composition selon l'invention a permis une réduction importante du biofilm (courbe D1, 10³ CFU) par rapport à une solution de soude (courbe C1, 10⁵ CFU). La composition selon l'invention est donc plus efficace qu'un traitement de référence (solution de soude) sur un biofilm résistant à l'arrachement mécanique. La FIG. 4 présente plusieurs images de la surface d'un substrat prises au microscope à épifluorescence après l'application des différentes solutions de traitement à 300 l/h. L'image (A4) montre la surface du substrat soumis à une solution d'eau, l'image (B4) montre la surface du substrat soumis à une solution de soude à 0,5% et l'image (C4) montre la surface du substrat soumis à une solution de la composition selon l'invention. Ces données montrent clairement que la composition selon l'invention a éliminé la totalité de la matrice des biofilms (coloration diffuse), ne laissant sur les surfaces que des cellules isolées ou de petits groupes de cellules réparties sur une seule couche, non protégées par une matrice. La solution de soude n'a quant à elle pas permis l'élimination complète de la matrice (coloration diffuse), qui protège toujours partiellement les cellules résiduelles. Une phase de désinfection ultérieure sera donc beaucoup plus efficace suite à l'application d'une solution de la composition selon l'invention.

### Test avec la souche de Bacillus mycoïdes

Le graphique représenté à la FIG. 2 montre la quantité de biofilm présente sur la paroi du cylindre après l'application de solutions de traitements suivant les conditions énoncées ci-dessus. Les surfaces du substrat ont été recouvertes d'un biofilm formé par la souche de *Bacillus mycoïdes*. La quantité de biofilm présente avant le traitement est repris à la courbe (A2). Cinq solutions ont ensuite été comparées : une solution comprenant uniquement de l'eau (B2), une solution de soude à 0,5% (C2), une solution de la composition selon l'invention (D2 et F2), et une solution de soude à 2% (E2). Les solutions A2-D2 ont été appliquées à un débit de 300 l/h tandis que les solutions E2-F2 ont été appliquées à un débit de 600 l/h. Le biofilm de *Bacillus mycoïdes* est relativement sensible à l'arrachement mécanique, sa quantité étant diminué de 90% par l'application de la solution (B2). L'application de la solution de la composition selon l'invention (D2 et F2) a montré des performances équivalentes à la solution de soude (C2 et E2) que le débit soit de 300 l/h ou de 600 l/h. La FIG. 5 représente plusieurs images de la surface du substrat prises au microscope à épifluorescence après l'application des différentes solutions de traitement à 300 l/h. L'image (A5) montre la surface du substrat soumis à une solution d'eau, l'image (B5) montre la surface du substrat soumis à une solution de soude à 0,5% et l'image (C5) montre la surface du substrat soumis à une solution de la composition selon l'invention. Ces données montrent clairement que la composition selon l'invention a éliminé la quasi-totalité de la matrice des biofilms, ne laissant que des cellules isolées ou de petits groupes de cellules réparties sur une seule couche, non protégées par une matrice. La solution de soude n'a pas permis une élimination plus efficace du biofilm. L'action postérieure d'une solution désinfectante sera donc plus efficace si une solution de la composition selon l'invention a précédemment été utilisée.

### Test avec la souche de Bacillus cereus

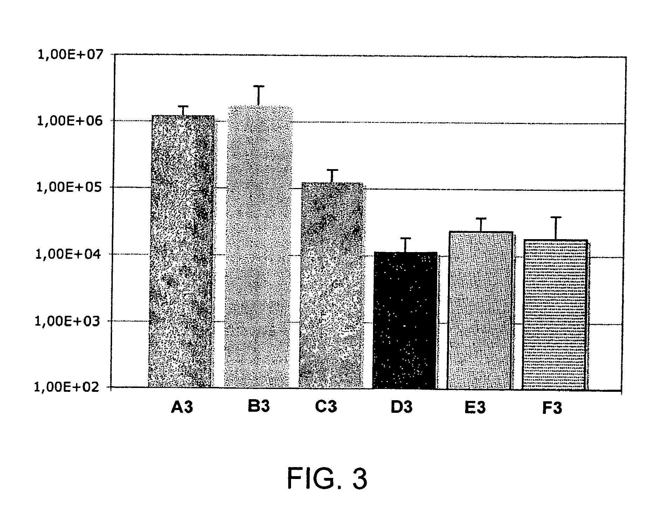

Le graphique représenté à la FIG. 3 montre la quantité de biofilm présente sur la paroi du cylindre après l'application de solutions de traitements suivant les conditions énoncées ci-dessus. Les surfaces du substrat ont été recouvertes d'un biofilm formé par la souche de *Bacillus cereus.* La quantité de biofilm présente avant le traitement est repris à la courbe A3. Cinq solutions ont été comparées : une solution comprenant uniquement de l'eau (B3), une solution de soude à 0,5% (C3), une solution de la composition selon l'invention (D3 et F3), et une solution de soude à 2% (E3). Les solutions B3, C3, D3 ont été appliquées à un débit de 300 l/h tandis que les solutions E3 et F3 ont été appliquées à un débit plus élevé (600l/h). Un débit plus élevé influence l'arrachement mécanique. Le biofilm de *Bacillus cereus* résiste totalement à l'arrachement mécanique (courbe A3 et B3). La composition selon l'invention a montré une plus grande efficacité à un débit de 300 l/h par rapport à une solution de soude à 0,5% (courbe C3) et même à un débit de 600 l/h par rapport à une solution de soude plus concentrée (courbe E3). La FIG. 6 représente plusieurs images de la surface d'un substrat prises au microscope à épifluorescence après l'application des différentes solutions de traitement. L'image (A6) montre la surface du substrat soumis à une solution d'eau, l'image (B6) montre la surface du substrat soumis à une solution de soude à 0,5% et l'image (C6) montre la surface du substrat soumis à une solution de la composition selon l'invention. Ces images montrent de nouveau que la composition selon l'invention élimine la quasi-totalité de la matrice des biofilms, ne laissant que des cellules isolées ou de petits groupes de cellules réparties sur une seule couche, non protégées par une matrice. La solution de soude ne permet pas une élimination efficace du biofilm. La phase de désinfection du substrat suivant la phase d'élimination du biofilm sera donc plus efficace après l'application d'une solution de la composition selon l'invention lors de cette phase d'élimination.

Les différents tests effectués dans cet exemple démontrent l'efficacité de la composition selon l'invention pour l'élimination d'un biofilm sur un substrat. La composition selon l'invention se révèle être plus efficace qu'une solution de soude couramment employée dans l'art antérieur. La composition selon l'invention se montre également très efficace pour plusieurs types de biofilm issus de bactéries différentes. La composition selon l'invention apporte donc une solution efficace aux problèmes mentionnés dans l'art et connue de l'homme du métier.

### Exemple 2

Des tests ont également été réalisés sur deux lignes de production en industrie (industrie de production de beurre et de margarine). Des coupons en acier inoxydable (8*1cm) sont placés durant 15 jours dans des circuits de production présentant des contaminations sporadiques. Ces coupons sont donc soumis aux cycles de production et de nettoyage standard de ces installations. La présence de biofilm dans l'installation a été déterminée par le développement d'un biofilm sur les coupons. Une procédure de nettoyage en place spécifique et faisant intervenir la composition selon l'invention a été appliquée dans l'installation, en présence des coupons contaminés, et dont l'efficacité a été démontrée par l'élimination des biofilms formés sur les coupons. La présence et l'importance des biofilms sur les coupons ont été déterminées en laboratoire par coloration des coupons et comparaison de la coloration obtenue avec une échelle visuelle (méthode HYDROBIO®, BKG), ainsi que par observation au microscope optique (40x et 100x).

Deux protocoles de nettoyage ont été testés : le premier protocole a servi de référence et correspond en l'application de solutions connues dans l'art, le second protocole correspond en l'ajout d'une étape d'application d'une solution de la composition selon l'invention.

En particulier, le protocole I comprend les étapes suivantes : de nettoyage à l'eau, nettoyage alcalin, de rinçage, de sanitation (ou désinfection) et de rinçage final. Le protocole I a été appliqué toutes les 32 heures pendant une semaine. Le nettoyage à l'eau a été appliqué pendant 15 minutes à une température comprise entre 60 et 65°C. Le nettoyage alcalin permet de nettoyer les canalisations de la matière organique présente suite à un cycle de production. Cette étape a été réalisée avec une solution de soude à 3,5% qui a été appliquée pendant 2*15 minutes à une température comprise entre 70-75°C. L'étape de sanitation permet l'élimination des germes éventuellement encore présents mais non protégés par la matrice du biofilm. Cette étape comprenait l'application d'une solution de Deptil OX à 1,5% (HYPRED) pendant 2*15 minutes à une température comprise entre 20 et 25°C. La solution de Deptil OX contient un mélange d'acide peracétique et de peroxyde d'hydrogène. Les étapes de rinçage ont été effectuées pendant 15 minutes à une température comprise entre 20 et 25°C.

Le protocole II différait du protocole I en ce qu'il comprenait une étape de nettoyage spécifique avant l'étape de sanitation (ou désinfection). En outre, le protocole II n'a été appliqué qu'une seule fois. L'étape de nettoyage spécifique consistait en l'application d'une solution de la composition selon l'invention. Ce mélange a été appliqué pendant 30 minutes à une température comprise entre 40 et 45°C. Les résultats obtenus sont repris dans le tableau 1.

**Tableau 1**

| | Quantité de biofilm suivant le protocole I (g/m²) | Quantité de biofilm suivant le protocole II (g/m²) |
|---|---|---|
| Ligne de production 1 | 25 | < 5 |
| Ligne de production 2 | 25 à 35 | < 5 |

Le protocole I n'a pas permis de limiter et d'empêcher la croissance d'un biofilm malgré l'étape de sanitation (désinfection). Le biofilm qui s'est formé sur les coupons en acier inoxydable au coeur de l'installation est donc très résistant. L'application du protocole II incluant une étape de nettoyage spécifique avec une solution de la composition selon l'invention a permis une élimination beaucoup plus efficace de ce biofilm (sous la limite de détection) de ce biofilm. Il a ainsi démontré une efficacité supérieure du nettoyage spécifique qui apporte donc une solution innovante aux problèmes de l'art antérieur.

## Revendications

1. Composition pour l'élimination des biofilms présents sur un substrat, ladite composition comportant un composant détergent contenant un séquestrant et un composant enzymatique contenant au moins une protéase et au moins une laccase, ladite composition étant **caractérisée en ce que** ledit composant détergent contient en outre un mouillant et un dispersant et **en ce que** ledit composant enzymatique comporte en outre au moins une polysaccharidase.

2. Composition selon la revendication 1, **caractérisée en ce que** la composition est une solution ayant un pH compris entre approximativement 8 et 11.

3. Composition selon l'une quelconque des revendications 1 ou 2, **caractérisée en ce que** le composant enzymatique comprend une proportion en protéase comprise entre 10 et 50%, une proportion en laccase comprise entre 5 et 35% et une proportion de polysaccharidase comprise entre 5 et 20%.

4. Composition selon l'une quelconque des revendications 1 à 3, **caractérisée en ce que** la au moins une polysaccharidase est une alpha-amylase.

5. Composition selon l'une quelconque des revendications 1 à 4, **caractérisée en ce que** le composant détergent comprend une proportion de sequestrant comprise entre 1 et 10%, une proportion de dispersant comprise entre 1 et 10% et une proportion de mouillant comprise entre 1 et 15%.

6. Méthode pour l'élimination des biofilms présents sur un substrat **caractérisée en ce qu'**elle comporte les étapes suivantes de :
a) mise à disposition d'un composant détergent contenant un séquestrant, un dispersant et un mouillant; et d'un composant enzymatique contenant au moins une protéase, au moins une laccase, et au moins une polysaccharidase,
b) mise en solution ou dilution du composant détergent dans l'eau,
c) mise en solution du composant enzymatique dans la solution formée à l'étape b) pour former la solution de ladite composition selon les revendications 1 à 5,
ou b') mise en solution ou dilution du composant enzymatique dans l'eau,
c') mise en solution du composant détergent dans la solution formée à l'étape b') pour former la solution de ladite composition selon les revendications 1 à 5,
d) application de la solution de ladite la composition formée à l'étape c) ou c') sur le substrat pendant une période de 15 minutes à 4 heures.

7. Méthode selon la revendication 6, **caractérisée en ce que** la méthode comprend en outre une étape ultérieure d'application d'un biocide sur le substrat.

8. Méthode selon la revendication 6 ou 7, **caractérisée en ce que** le pH de la solution de ladite composition est compris entre 8 et 11.

9. Utilisation d'une composition selon les revendications 1 à 5 pour l'élimination de biofilms présents sur un substrat.

10. Utilisation selon la revendication 9, d'une composition selon les revendications 1 à 5 pour le nettoyage des sols et des surfaces, pour le nettoyage en place ou le trempage.

11. Utilisation selon la revendication 10 dans laquelle ledit nettoyage par trempage est utilisé pour nettoyer du matériel chirurgical.

12. Trousse pour l'élimination de biofilms sur un substrat **caractérisée en ce qu'**elle comprend au moins un échantillon d'un composant détergent en solution ou sous forme solide contenant un séquestrant, un dispersant et un mouillant, et au moins un échantillon d'un composant enzymatique en solution ou sous forme solide contenant au moins une protéase, au moins une laccase et au moins une polysaccharidase.

13. Trousse selon la revendication 12, **caractérisée en ce que** l'échantillon du composant enzymatique est une solution aqueuse dont le pH est compris entre 8 et 10.

14. Trousse selon la revendication 12 ou 13, **caractérisée en ce que** l'échantillon du composant détergent est une solution aqueuse dont le pH est compris entre 12,8 et 13,8.

## Patentansprüche

1. Zusammensetzung zur Entfernung von Biofilmen, die sich auf einem Substrat befinden, wobei die Zusammensetzung einen reinigenden Bestandteil aufweist, der ein Komplexierungsmittel enthält, sowie einen enzymatischen Bestandteil, der mindestens eine Protease und mindestens eine Laccase enthält, wobei die Zusammensetzung **dadurch gekennzeichnet ist, dass** der reinigende Bestandteil darüber hinaus ein Netzmittel und ein Dispergiermittel enthält und dass der enzymatische Bestandteil darüber hinaus mindestens eine Polysaccharidase enthält.

2. Zusammensetzung nach Anspruch 1, **dadurch gekennzeichnet, dass** die Zusammensetzung eine Lösung ist, die einen pH-Wert im Bereich von näherungsweise 8 bis 11 hat.

3. Zusammensetzung nach einem beliebigen der Ansprüche 1 oder 2, **dadurch gekennzeichnet, dass** der enzymatische Bestandteil einen Proteaseanteil im Bereich von 10 bis 50 %, einen Laccaseanteil im Bereich von 5 bis 35 % und einen Polysaccharidaseanteil im Bereich von 5 bis 20 % umfasst.

4. Zusammensetzung nach einem beliebigen der Ansprüche 1 bis 3, **dadurch gekennzeichnet, dass** es sich bei der mindestens einen Polysaccharidase um eine alpha-Amylase handelt.

5. Zusammensetzung nach einem beliebigen der Ansprüche 1 bis 4, **dadurch gekennzeichnet, dass** der reinigende Bestandteil einen Komplexierungsmittelanteil im Bereich von 1 bis 10 %, einen Dispergiermittelanteil im Bereich von 1 bis 10 % und einen Netzmittelanteil im Bereich von 1 bis 15 % umfasst.

6. Verfahren zur Entfernung von Biofilmen, die sich auf einem Substrat befinden, **dadurch gekennzeichnet, dass** es die folgenden Schritte aufweist:
a) Bereitstellen eines reinigenden Bestandteils, der ein Komplexierungsmittel, ein Dispergiermittel und ein Netzmittel enthält; und eines enzymatischen Bestandteils, der mindestens eine Protease, mindestens eine Laccase und mindestens eine Polysaccharidase enthält,
b) Inlösungbringen oder Verdünnen des reinigenden Bestandteils in Wasser,
c) Inlösungbringen des enzymatischen Bestandteils in der Lösung, die in Schritt b) gebildet wurde, um die Lösung der Zusammensetzung gemäß den Ansprüchen 1 bis 5 zu bilden,
oder
b') Inlösungbringen oder Verdünnen des enzymatischen Bestandteils in Wasser,
c') Inlösungbringen des reinigenden Bestandteils in der Lösung, die in Schritt b') gebildet wurde, um die Lösung der Zusammensetzung gemäß den Ansprüchen 1 bis 5 zu bilden,
d) Aufbringen der Lösung der Zusammensetzung, welche in Schritt c) oder c') gebildet wurde, auf das Substrat während einer Zeitdauer von 15 Minuten bis 4 Stunden.

7. Verfahren nach Anspruch 6, **dadurch gekennzeichnet, dass** das Verfahren darüber hinaus im weiteren Verlauf einen Schritt des Aufbringens eines Biozids auf das Substrat umfasst.

8. Verfahren nach Anspruch 6 oder 7, **dadurch gekennzeichnet, dass** der pH-Wert der Lösung der Zusammensetzung im Bereich von 8 bis 11 liegt.

9. Verwendung einer Zusammensetzung gemäß den Ansprüchen 1 bis 5 zur Entfernung von Biofilmen, die sich auf einem Substrat befinden.

10. Verwendung nach Anspruch 9, einer Zusammensetzung nach den Ansprüche 1 bis 5, um Böden und Oberflächen zu reinigen, um eine Reinigung vor Ort oder ein Eintauchen vorzunehmen.

11. Verwendung nach Anspruch 10, wobei die Reinigung durch Eintauchen zur Anwendung kommt, um chirurgische Geräte zu reinigen.

12. Anwendungseinheit zur Entfernung von Biofilmen, die sich auf einem Substrat befinden, **dadurch gekennzeichnet, dass** sie mindestens eine Probe eines reinigenden Bestandteils in Lösung oder in fester Form umfasst, welcher ein Komplexierungsmittel, ein Dispergiermittel und ein Netzmittel enthält, und mindestens eine Probe eines enzymatischen Bestandteils in Lösung oder in fester Form umfasst, welcher mindestens eine Protease, mindestens eine Laccase und mindestens eine Polysaccharidase enthält.

13. Anwendungseinheit nach Anspruch 12, **dadurch gekennzeichnet, dass** es sich bei der Probe des enzymatischen Bestandteils um eine wässrige Lösung handelt, deren pH-Wert im Bereich von 8 bis 10 liegt.

14. Anwendungseinheit nach Anspruch 12 oder 13, **dadurch gekennzeichnet, dass** es sich bei der Probe des reinigenden Bestandteils um eine wässrige Lösung handelt, deren pH-Wert im Bereich von 12,8 bis 13,8 liegt.

## Claims

1. Composition for removing biofilms present on a substrate, said composition comprising a detergent component containing a sequestrant and an enzymatic component containing at least one protease and at least one laccase, said composition being **characterized in that** said detergent component futher comprises a wetting agent and a dispersant and **in that** said enzymatic component futher comprises at least one polysaccharidase.

2. Composition according to claim 1, **characterized in that** the composition is a solution having a pH of between approximately 8 and 11.

3. Composition according to claims 1 or 2, **characterized in that** the enzymatic component comprises a proportion of protease of between 10 and 50 %, a proportion of laccase of between 5 and 35 % and a proportion of polysaccharidase(s) of between 5 and 20 %.

4. Composition according to any of claims 1 to 3, **characterized in that** said at least one polysaccharidase is an alpha-amylase.

5. Composition according to any of claims 1 to 4, **characterized in that** the detergent component comprises a proportion of sequestrant of between 1 and 10 %, a proportion of dispersant of between 1 and 10 % and a proportion of wetting agent of between 1 and 15 %.

6. Method for removing biofilms present on a substrate, **characterized in that** it comprises the following steps of:
- a) providing a detergent component containing a sequestrant, a dispersant and a wetting agent; and an enzymatic component containing at least one protease, at least one laccase and at least one polysaccharidase,
- b) placing or dilution of the detergent component in water,
- c) placing the enzymatic component in solution in the solution formed at step b) to form the solution of said composition according to any of claims 1 to 5,
or -b') placing or dilution of the enzymatic component in water,
-c') placing the detergent component in solution in the solution formed at step b') to form the solution of said composition according to any of claims 1 to 5,
- d) applying the solution of said composition formed at step c) or c') to the substrate for a period of time from 15 minutes and 4 hours.

7. Method according to claim 6, **characterized in that** it further comprises a subsequent step to apply a biocide to the substrate.

8. Method according to claims 6 or 7, **characterized in that** the pH of the solution of said composition is between 8 and 11.

9. Use of a composition according to any of claims 1 to 5 to remove biofilms present on a substrate.

10. Use according to claim 9, of a composition according to any of claims 1 to 5 to clean floors and surfaces, for cleaning by clean-in-place or by immersion.

11. Use according to claim 10 wherein said cleaning by immersion is used to clean surgical equipment.

12. Kit to remove biofilms present on a substrate **characterized in that** it comprisesat least one sample of a detergent component in solution or in solid form containing a sequestrant, a dispersant and a wetting agent, and at least one sample of an enzymatic component in solution or in solid form containing at least one protease, at least one laccase and at least one polysaccharidase.

13. Kit according to claim 12, **characterized in that** the sample of the enzymatic component is an aqueous solution having a pH between 8 and 10.

14. Kit according to claim 12 or 13, **characterized in that** the sample of the detergent component is an aqueous solution having a pH between 12.8 and 13.8.
